(19) Europäisches Patentamt
European Patent Office
Office européen des brevets

(11) EP 1 460 561 A1

(12) **EUROPEAN PATENT APPLICATION**
published in accordance with Art. 158(3) EPC

(43) Date of publication:
**22.09.2004 Bulletin 2004/39**

(21) Application number: **02793448.8**

(22) Date of filing: **27.12.2002**

(51) Int Cl.[7]: **G06F 17/30**, C12N 15/00

(86) International application number:
**PCT/JP2002/013815**

(87) International publication number:
**WO 2003/056460 (10.07.2003 Gazette 2003/28)**

(84) Designated Contracting States:
**AT BE BG CH CY CZ DE DK EE ES FI FR GB GR IE IT LI LU MC NL PT SE SI SK TR**
Designated Extension States:
**AL LT LV MK RO**

(30) Priority: **27.12.2001 JP 2001396194**

(71) Applicant: **TOUDAI TLO, Ltd.**
**Tokyo 113-0033 (JP)**

(72) Inventors:
- **Morishita, Shinichi**
  **Yokohama-shi, Kanagawa 224-0029 (JP)**
- **YAMADA, Tomoyuki**
  **Bunkyo-ku, Tokyo 113-0033 (JP)**

(74) Representative: **Zimmermann, Gerd Heinrich et al**
**Zimmermann & Partner,**
**P.O. Box 33 09 20**
**80069 München (DE)**

(54) **METHOD OF CALCULATING OCCURRENCE FREQUENCY OF SEQUENCE, METHOD OF CALULATING DEGREE OF ISOLATION AND METHOD OF ESTIMATING DEGREE OF ADEQUACY FOR PRIMER**

(57) It is intended to support a unique design of a primer. To calculate an indication showing the occurrence frequency of a sequence in a genome sequence, the occurrence frequencies of partial sequences having a definite length in the genome sequences are calculated. Then the occurrence of frequency of each partial sequence of the definite length is stored in an incidence/isolation degree table (16). Concerning each partial sequence of the definite length, a degree of isolation i, which means that j mutation indicating the conversion of j bases (j=<i-1) does not occur in the genome sequence but i mutation indicating the conversion of i bases occurs in the genome sequence, is calculated. Then the degrees of isolation of the partial sequences of the definite length are stored in the incidence/isolation degree table (16). In a visualization processing portion (18), respective bases are clolored in a definite manner based on the occurrence of frequency and/or the degree of isolation of each base to give an image showing the genome sequence.

FIG. 1

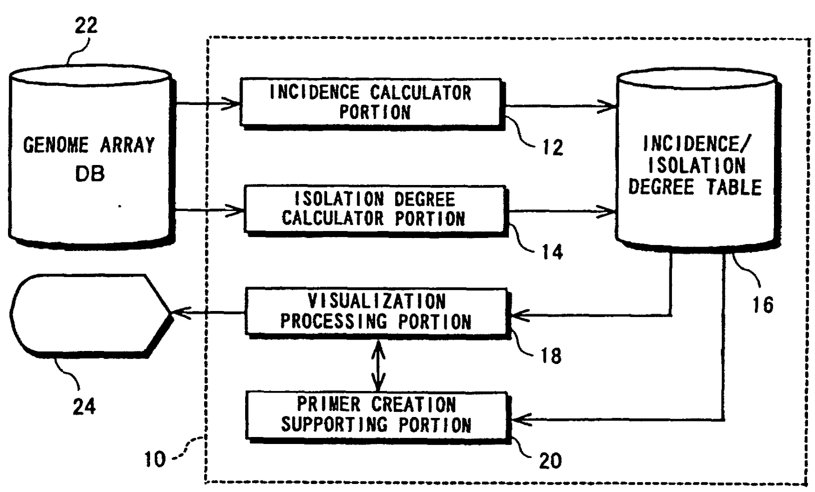




Printed by Jouve, 75001 PARIS (FR)

## Description

Technical Field to which the Invention Belongs

The present invention relates to a method for supporting primer selection.

Background Art

While many primer design methods have been proposed in the past, it is currently difficult to design a primer, which is annealed only in one place. By calculating incidences of combinations of all possible alkali arrays having shorter arrays (K-tupples) than an EST array registered with a database, for example, by calculating incidences of $4^8$(65536) kinds of 8-mer alkali arrays, an array with a high incidence and an array with a low incidence can be found. This kind of method is disclosed in "Nucleic Acids Res. 19 3887-3891 (R. Griffais, P.M. Andre and M. Thibon: 1991)", for example.

However, since several famous EST databases have many similar arrays contributed by many researchers, incidences of the arrays cannot be discussed as they are.

In order to design a primer sandwiching genes, an array in a promoter region is required. Therefore, the primer cannot be designed only with an EST database, which is a problem.

Even though DNA polymerase is oligonucleotide with several mismatches, it is known that DNA polymerase can be recognized as a primer (refer to "Molecular Biology Vol. 28, No. 5, Part I 661-663 (L.B.D' Yachenko, A.A. Chenchick, G.L. Khaspekov, A.O. Tatarenko and R. Sh. Bibilashvili: 1994")), for example. However, primer design methods proposed in the past do not consider genomewide mismatch tolerance. Furthermore, in the past primer design methods, a mismatch tolerance is searched in a database after an alkali array of a given primer is determined. Therefore, the search takes time, which is another problem.

It is an object of the invention to support unique primer design.

Disclosure of the Invention

According to the invention, an incidence of an array with a predetermined length (N-mer) in a genome array is counted and is evaluated by introducing an isolation degree, which is another aspect of array uniqueness, as a value for evaluating the mismatch tolerance. The isolation degree is defined as a minimum hamming distance between arrays, for example. By introducing the isolation degree, the uniqueness of an alkali array can be categorized more precisely.

More specifically, the object of the invention can be achieved by a method for calculating an indicator indicating an incidence of an array in a genome array, the method characterized by the steps of calculating incidences of partial arrays with a predetermined length in the genome array, and storing the incidences relating to the partial arrays with the predetermined length in an incidence table.

The step of storing in the incidence table desirably has the steps of omitting the storage into the incidence table for partial arrays with the incidence of zero (0), and using second partial arrays having a shorter second predetermined length than the predetermined length and storing in a second table a position in the incidence table of the partial arrays with the predetermined length including the second partial array from the beginning. Thus, the memory capacity and processing time can be reduced.

The object of the invention can be achieved by a method for calculating an indicator indicating an isolation degree of an array in a genome array, the method characterized by including the steps of calculating an isolation degree i by which j mutation(s) (j=1,2, ..., i-1) referring to the conversion of j alkali(s) of each of partial arrays with a predetermined length do/does not appear in the genome array but i mutation(s) referring to the conversion of i alkalis appear(s) in the genome array; and storing in an isolation degree table the isolation degree with respect to the partial arrays with the predetermined length.

A unique part can be identified easily in a genome array by using the incidence and/or isolation degree, and more unique primer can be designed.

According to a preferred embodiment, the step for calculating the isolation degree has the steps of judging whether or not k mutation(s) referring to the conversion of k alkali(s) of the partial array with the predetermined length exist(s) in the partial array with the predetermined length with reference to an incidence table storing an incidence in a genome array with respect to each of the partial arrays with the predetermined length, when the k mutation(s) exist(s), determining k as an isolation degree, when the k mutation(s) does/do not exist, incrementing k and repeating the step of judging the presence of the k mutation(s).

According to another preferred embodiment, the step of calculating the isolation degree has the steps of judging, by using second partial arrays having a shorter second predetermined length than the predetermined length and with reference to a second table storing a position, in the incidence table, of the partial arrays with the predetermined length including the second partial array from the beginning, whether the k mutation(s) with the predetermined length exist(s) in which k alkali(s) at a position away from the beginning of the partial array with the predetermined length by a second predetermined length is/are converted, when the k mutation(s) exist(s), finding a hamming distance between the k mutation(s) and the array with the predetermined length, when the minimum value of the hamming distance is k, determining the k as an isolation degree thereof, when the minimum value is larger than k, repeating the step of incrementing k and judging by using the presence of the k mutation(s) with the predetermined length and the minimum value of the hamming distance.

According to another preferred embodiment, the method includes the step of judging the appearance in the genome array based on whether the incidence in the genome array is equal to or lower than n. When a genome array is not organized or when a same genetic array actually appears in a genome array only twice, an isolation degree extended based on whether the incidence is equal to or lower than three or not (second isolation degree) is obtained. Thus, primer design can be achieved for a partial array which appears in a genome array three times or below but is similar to no other arrays in the genome array.

According to another preferred embodiment, a method for calculating an indicator indicating an isolation degree of a genome array includes the steps of calculating a shortest partial array by which a partial array starting from the $k^{th}$ letter of a partial array with a predetermined length no longer appears in a genome array, and calculating the maximum number m of partial array uniquely included in the partial array and handling the m as an indicator indicating an isolation degree thereof by considering the m as the lower bounds of the isolation degree.

The absence of similar arrays is assured by the lower bound of the isolation degree for primer selection instead of an accurate isolation degree of a longer array (such as a 50-mer array in a human genome array). For example, when a lower bound of the isolation degree is "7", arrays having 90% similarity or more do not exist in a 50-mer array. Thus, the absence of an array having 60% similarity or more does not have to be proved accurately. The knowledge of the absence of arrays having 90% similarity or more is enough as an indicator for the primer selection.

In the embodiment, the step of judging whether the partial array appears or not may be performed based on whether the incidence in the genome array is equal to or lower than n.

The object of the invention can be also achieved by a method for calculating a first indicator indicating an eligibility for a primer of an array including a given alkali with respect to alkalis in a genome array by using an incidence table created by using the method, characterized by including the steps of identifying a same number of arrays including the alkali as a predetermined length with respect to each of alkalis included in a genome array, identifying an incidence relating to each of the identified arrays with reference to the incidence table, and calculating the first indicator based on a total sum of the identified incidences.

The object of the invention can be also achieved by a method for calculating a second indicator indicating an eligibility for a primer of an array including a given alkali with respect to alkalis in a genome array by using an isolation degree table created by using the method, characterized by including the steps of identifying a same number of arrays including the alkali as a predetermined length with respect to each of alkalis included in a genome array, identifying an isolation degree relating to each of the identified arrays with reference to the isolation degree table, and calculating the second indicator based on a total sum of the identified isolation degrees.

The object of the invention can be also achieved by a method for calculating a third indicator indicating an eligibility, for a primer, of an array including a given alkali with respect to alkalis in a genome array by using an incidence table and isolation degree table created by using the method, characterized by including the steps of identifying a same number of arrays including the alkali as a predetermined length with respect to each of alkalis included in a genome array, identifying an incidence relating to each of the identified arrays with reference to the incidence table, calculating a first indicator based on a total sum of the identified incidences, identifying an isolation degree relating to each of the identified arrays with reference to the isolation degree table, and calculating a second indicator based on a total sum of the identified isolation degrees.

By using these methods, indicators at an alkali level in a genome array can be obtained, and design of a more unique primer can be supported.

The object of the invention is also achieved by a method characterized by including the steps of assigning, based on an indicator obtained by using the method, a different display form in accordance with a value or range of the indicator, and creating an image representing each alkali in a genome array in accordance the assigned display form. For example, the display form may be a color.

The object of the invention can be also achieved by a program for operating a computer for calculating an indicator indicating an incidence of an array in a genome array and being readable by the computer, the program causing the computer to perform the steps of calculating incidences of partial arrays with a predetermined length in the genome array, and storing the incidences relating to the partial arrays with the predetermined length in an incidence table.

The object of the invention can be also achieved by a program for operating a computer for calculating an indicator indicating an isolation degree of an array in a genome array and being readable by the computer, the program causing the computer to perform the steps of calculating an isolation degree i by which j mutation(s) (j=1,2, ..., i-1) referring to the conversion of j alkali(s) of each of partial arrays with a predetermined length do/does not appear in the genome array but i mutation(s) referring to the conversion of i alkalis appear (s) in the genome array, and storing in an isolation degree table the isolation degree with respect to the partial arrays with the predetermined length.

Brief Description of the Drawings

Fig. 1 is a block diagram illustrating an overview of a primer design support system according to an embodiment of the invention.
Fig. 2A is a diagram for describing incidences of genome arrays according to the embodiment; and
Fig. 2B is a diagram for describing a first indicator based on incidences.
Fig. 3 is a diagram for describing an isolation degree according to the embodiment.
Fig. 4 is a flowchart illustrating processing for an incidence calculation according to the embodiment.
Figs. 5A to 5C are diagrams for describing tables relating to incidences according to the embodiment.
Fig. 6 is a flowchart illustrating processing for isolation degree calculation according to the embodiment.
Fig. 7 is a diagram for describing colors to be assigned in visualization processing according to the embodiment.
Fig. 8 is a graph showing a maximum height of a human genome array, which is calculated according to a second embodiment.
Fig. 9 is a flowchart showing an overview of processing to be performed in a design support apparatus according to the second embodiment.

Preferred Mode for Carrying Out the Invention

Embodiments of the invention will be described below with reference to attached drawings. Fig. 1 is a block diagram illustrating an overview of a primer design support system according to an embodiment of the invention. As shown in Fig. 1, the primer design support system 10 has an incidence calculator portion 12, an isolation degree calculator portion 14, an incidence/isolation degree table 16, a visualization processing portion 18, and a primer creation supporting portion 20. The incidence calculator portion 12 calculates an incidence of a partial array having a predetermined length (N-mer array) in a given genome array with reference to the genome array to be used for primer design. The isolation degree calculator portion 14 calculates an isolation degree of each partial array with respect to the genome array as described later. The incidence/isolation degree table 16 stores an incidence and isolation degree relating to each partial degree. The visualization processing portion 18 performs processing required for visualizing and displaying the genome array with reference to the incidence/isolation degree table 16. The primer creation supporting portion 20 performs processing of selecting an area to be used as a primer with reference to an image displayed on a screen of a display apparatus 24 by the processing by the visualization processing portion 18 in response to a manipulation on an input apparatus (not shown) by a user.

The primer design support system 10 can be implemented by installing a design support program to a computer. According to this embodiment, a genome array is read from a genome array database (DB) 22. The genome array DB 22 may be on a hard disk of the personal computer or may be loaded in a server spaced from the personal computer. In the latter case, the personal computer may access the server over a network such as a LAN and Internet and refer to data in the genome array DB.

Before describing processing by the primer design support system 10, a principle of the invention will be briefly described below.

Fig. 2A is a diagram for describing incidences of a genome array. Here, an extremely short genome array, "ATATGGGATC", is used, and 2-mer arrays are considered as partial arrays thereof. As shown in Fig. 2A, a 2-mer array, "AT", appears three times in the genome array, and a 2-mer array, "GG", appears twice in the genome array. Other 2-mer arrays ("TA", "TG", "GA", and "TC") appear once, and still other arrays (such as "AA" and "AC") do not appear.

In this way, the incidence calculator portion 12 calculates how many times each of 2-mer arrays appears (incidence) in a genome array.

Next, an isolation degree will be described with reference to the same genome array and 2-mer arrays as those of the example in Fig. 2A. Fig. 3 is a diagram for describing an isolation degree of each of the 2-mer arrays (partial arrays) with respect to the genome array. An isolation degree is defined herein as a minimum hamming distance between arrays. In other words, the isolation degree of the partial array is "n" where a partial array in which alkalis at n positions are replaced by other alkalis appears in the genome array though a partial array in which alkalis at i positions (i<n) are replaced by other alkalis does not appear in the genome array.

In the example shown in Fig. 3, those resulting from replacement of an alkali at one position of the partial array "AT" (one mutation, refer to the reference numeral 302), that is, "AA", "AG", "AC", "TT", "GT" and "CT" do not appear in the genome array (refer to the reference numeral 300). On the other hand, the ones underlined in Fig. 3 ("TA", "TC", "GA" and "GG") of those resulting from replacement of alkalis at two positions thereof (two mutations, refer to the reference numeral 303), that is, "TA" , "TG", "TC", "GA", "GG", "GC", "CA", "CG" and "CC" appear in the genome array (refer to the reference numeral 300). Therefore, the isolation degree of the partial array "AT" is "2".

Similarly, since one mutation of each of the other partial arrays "TA", "TG", "GG", "GA" and "TC" occurs in the genome array, the isolation degrees is "1".

According to this embodiment, an incidence and an isolation degree are calculated by using an 18-mer partial array, for example. Fig. 4 is a flowchart showing an overview of processing to be performed for calculating an incidence according to this embodiment. As shown in Fig. 4, the incidence calculator portion 12 selects an N-mer array (such as an 18-mer array) (step 401) and scans a genome array obtained from the genome array DB 22 (step 402). Thus, positions where the N-mer arrays appear can be located in the genome array, and the incidences can be obtained by counting them (step 403).

The incidence calculator portion 12 stores the incidence and so on, which are obtained at the step 403, in the incidence/isolation degree table 16 by using a given N-mer array to be processed as a key (step 404). The above-described processing is performed on all possible N-mer arrays (refer to a step 405). Thus, a table can be created.

In reality, according to this embodiment, by preventing N-mer arrays with the incidence of 0 from appearing, the size of the table can be reduced. For example, in the example shown in Fig. 2A, the table including incidences is originally as shown in Fig. 5A. However, according to this embodiment, the size is reduced as shown in Fig. 5B. The table may be called "map size table".

Furthermore, by limiting a part to be referred in a table, the speed of processing is increased. For example, in the example shown in Fig. 2A, by using a 2-mer array itself as a key, a table 501 as shown in Fig. 5B can be obtained. However, in order to increase the speed of processing, a sub-table 502 may be provided in which an alkali array having a shorter length (which may be called "hash size") is used as a key as shown in Fig. 5B. Thus, the approximate position thereof in the table can be located. This kind of sub-table may be called "hash-size table". When a map-size table for N=18 is used, the hash size ≤ 14 desirably, for example.

Next, the isolation degree calculator portion 14 calculates the isolation degree by referring to the map-size table and sub-table (hash-size table) in which the N-mer arrays and incidences as a result of the processing in Fig. 4 correspond to each other. Fig. 6 is a flowchart describing processing of calculating an isolation degree.

First, the isolation degree calculator portion 14 selects an N-mer array first (step 601) and initializes "i" indicating the number of mutations to 1 (step 602). Next, another N-mer array, which is i mutation(s) of the N-mer array, is selected (step 603). The isolation degree calculator portion 14 refers to the hash-size table (step 604) and judges whether or not the other N-mer array, that is, the first alkali array with a hash-size length appears in the genome array (step 605).

If No at the step 605 and if another N-mer array having i mutation(s) remains (No at a step 606), the processing at the steps 603 to 605 is performed on the other N-mer array. Alternatively, if the presence of appearance of all other N-mer arrays having i-mutation(s) in the genome array is judged, i is incremented (step 607). Then, the same processing (steps 603 to 606) is repeated for the incremented i mutation(s).

Here, a technique of identifying N-mer arrays having i-mutation(s) and referring to the table (steps 603 and 604) will be described. According to this embodiment, in reality, at the step 603, an array in a hash-size (hash array) in which a predetermined number of alkalis from the beginning in the N-mer array selected at the step 601 are the same is identified. Then, an array in which i alkalis of the alkalis are converted is created, and how many N-mer arrays including the hash array in the beginning exist is obtained with reference to the hash-size table and, then, the map-size table. Thus, a list of the N-mer arrays can be obtained.

Next, the isolation degree calculator portion 14 calculates a hamming distance between each of the resulting N-mer arrays and the N-mer array to be processed (the one selected at the step 601) and judges whether the minimum value of the hamming distance is equal to i or not (step 608). This is because no calculation is required for the rest if the minimum value is i since all of the listed N-mer arrays include i-mutation(s) of the N-mer array to be processed.

If judged as Yes at the step 608, i is stored in the table as the isolation degree of the N-mer array to be processed. On the other hand, if judged as No at the step 608, and if the minimum value of the hamming distance is larger than i, (i+x) mutations (x≥1) exist. Thus, i is incremented, and the steps 603 and 604 are repeated. Then, it is judged whether the minimum value of the hamming distance between the listed N-mer arrays and the N-mer array to be processed is equal to i or not. Therefore, a large amount of processing time is not required, and the isolation degree of each of the N-mer arrays can be calculated.

The visualization processing portion 18 visualizes alkalis in the genome array and creates an image by using the incidence/isolation degree table 16 resulting from the processing in Fig. 4 and Figs. 5A to 5C. The technique will be described below. For example, according to this embodiment, an indicator relating to an incidence of each alkali is obtained based on the incidence of the N-mer array. Fig. 2B is a diagram illustrating a method of calculating an indicator of an element "A (see the arrow 212)" in a genome array (the reference numeral 211) when N=6. Here, it is assumed that N arrays include the element "A", and a first indicator is obtained by calculating (a total sum of incidences of N arrays)/N.

In the example in Fig. 2B, 6-mer arrays "ATGCCA", "TGCCAG", "GCCAGT", "CCATGC", "CAGTCA" and "AGTCAG" appear eight times, twice, three times, once, three times and four times, respectively, in the genome array. Therefore, the indicator to obtain is (8+2+3+1+3+4)/6. The speed of the indicator calculation can be increased by referring to the incidence/isolation degree table.

An isolation degree can be obtained similarly. A second indicator relating to an isolation degree of each alkali can be obtained. Also in this case, the speed of the indicator calculation can be increased with reference to the incidence/isolation degree table.

For example, the visualization processing portion 18 determines a color to be assigned to each alkali for displaying the genome array based on the first indicator and the second indicator or a third indicator, which is a combination of the first indicator and the second indicator. According to this embodiment, as the incidence decreases, that is, as the value of the first indicator decreases, the possibility that the array containing the alkali is a unique primer increases. On the other hand, as the isolation degree increases, that is, the value of the second indicator increases, the possibility that the array containing the alkali is a unique primer increases. By using these facts, it may be set that, as the value of the third indicator increases where the third indicator=(second indicator/first indicator), the possibility that the array containing the alkali is a unique primer increases.

As shown in Fig. 7, the visualization processing portion 18 defines that, as the value of the first indicator decreases, the level of coldness of the color increases while as the value increases, the level of warmness of the color increases. Alternatively, the visualization processing portion 12 defines that, as the value of the second indicator increases, the level of coldness of the color increases while as the value decreases, the level of warmness of the color increases. In accordance with the setting, the visualization processing portion 12 assigns a color to each alkali. Apparently, as the value of the third indicator increases, the level of coldness of the color increases, as shown in Fig. 7.

In this way, when an image colored in consideration with the incidence and/or isolation degree of each alkali in the genome array is displayed on the screen of the display apparatus 24, an operator can identify primer candidates, which may be more unique, with reference to the image. The user can intuitively find primer candidates, which may be unique, with reference to the color given to the genome array. The primer creation support portion 20 includes a tool for selecting the presence of the formation of a complementary chain in an array and/or a melting temperature and a tool (program), for avoiding an optimum GC content, a short repeated array and/or a palindrome array. Thus, processing required in accordance with an instruction from a user can be performed on a primer candidate selected by the user. Therefore, the user can design a predetermined primer.

According to this embodiment, alkalis in a genome array can be visualized based on incidences in the genome array of an array with a predetermined length (N) and an isolation degree of the array with the predetermined length with respect to the genome array. Therefore, a user can intuitively and visually check an array including a more unique alkali. During the calculation of an incidence and an isolation degree, a processing time required for the visualization is reduced by using the incidence/isolation degree table. Furthermore, a processing time for creating an isolation degree relating to the array with the predetermined length can be reduced by using a hash table relating to an array with a shorter length than N.

Apparently, the invention is not limited to the embodiment, and various changes and modifications may be made without departing from the spirit and scope of the invention. It will be understood that the changes and modifications fall within the spirit and scope of the invention.

For example, according to the embodiment, both of map-size table relating to an array with a predetermined length (N) and hash table relating to a shorter array are created for a table relating to incidences. By using them, an isolation degree relating to the array with the predetermined length can be calculated, and/or an incidence for creating an indicator can be identified, for example. However, the invention is not limited to these constructions. A map-size table may be only provided, and the processing may be performed by a so-called binary search.

In the embodiment, the map size is 18 (N=14), and the hash size ≤ 14. However, the sizes are not limited thereto. Apparently, tables relating to arrays having other sizes may be created.

Furthermore, an indicator for each alkali is not limited to the one according to the embodiment. The visualization technique based on an indicator is not limited to the one according to the embodiment, either.

While a different color is assigned in accordance with an indicator in the embodiment, the assignment is not limited thereto. A different lightness of grayscale may be assigned. Alternatively, a different display form may be assigned in accordance with an indicator.

Furthermore, according to the embodiment, the primer design support system includes the incidence calculator portion 12 and the isolation degree calculator portion 14 and creates a table indicating incidences and isolation degrees based on an array from the genome array DB 22. The created table is used by the visualization processing portion 18. However, all of them are not required. For example, a table may be created by a system including the incidence calculator portion 12 and the isolation degree calculator portion 14, and the table may be recorded in a recording medium such as a CD-ROM and a DVD-ROM. In this case, a system having the visualization processing portion 18 may read the recording medium and implement processing for assigning a different color in accordance with an indicator relating to a given alkali, for example.

According to the embodiment, the invention is applied for supporting design of a primer such as a PCR primer. However, the invention may be also applied for design of microarray oligonucleotide, array design for RNAi, array design for gene screening, and array design for genome typing. Therefore, the "primer" herein may include an oligomer array.

[Second Embodiment]

Next, a second embodiment of the invention will be described. Before describing a construction and processing of a system, a principle of the second embodiment will be described below. According to the second embodiment, a second isolation degree (that is, extended isolation degree) in which the concept of an isolation degree is extended is introduced, and various kinds of calculation are performed by using the second isolation degree. Again, an isolation degree will be described briefly, and the second isolation degree in which the isolation degree is extended will be described.

[Second Isolation Degree]

"G" refers to a genome array having a length |G| here. For example, for a human genome, |G| is equal to about 3 Gbp. A partial array "E" thereof is a genome array having a length |E|. Here, the genome array E is a short array. For example, when the partial array E of the genome array G appears in the genome array G only once, the isolation degree of "E" with respect to "G" is the minimum value of the number of mismatched alkalis as a result of the comparison between "E" and all of the partial arrays of "G" (where the original array is excluded). As the isolation degree increases, the possibility that E couples with a wrong place (inappropriate place) decreases.

A partial array from an "l"$^{th}$ letter to an "r"$^{th}$ letter in the genome array G is written as $G_{[l,r]}$. A hamming distance between an array S and an array T is written as $d_H(S,T)$. Therefore, the hamming distance $d_H(S,T)$ may be expressed as:

$$d_H(S,T) = |\{i|S_{[i,i]} \neq T_{[i,i]}, i=l, ..., k\}|$$

Here, the isolation degree isol(E,G) of the partial array E with respect to the genome array G may be defined as:

$$isol(E,G)=\min\{dH(E,G_{[i,i+k-1]}| \\ k=|E|, i=1, ..., |G|-k+1, E\neq G_{[i,i+k-1]}\}$$

For example, when the genome array S is "ATGCTGCGATCGTA" and the genome array T is "ATGTTGCGATCCTA", the hamming distance between the genome array S and the genome array T is "2". When the genome array G is the same as the array S and when the partial array E is an array "ATGCT" having the first five elements of the genome array S, isol(E,G) = 2.

Next, the extended second isolation degree will be described. When all of arrays with the length |E| included in the genome array G are sorted in order of increasing hamming distance with respect to the partial array E, the "n"$^{th}$ array is n-neighbor of the array E and is written as $neighbor_n(E,G)$. Here, the second isolation degree, that is, the extended isolation degree $isol_n(E,G)$ is defined as:

$$isol_n(E,G) = d_H (E, neighbor_n(E,G)).$$

The $isol_1(E,G)$ is the above-described isolation degree.

For calculating the second isolation degree, a suffix array is used. This will be described briefly. The array G[1,...,n]=G[1]G[2]...G[n] will be considered. Here, G[n]=$ is the largest end letter among other elements. The j$^{th}$ suffix of G is defined as G[j,...n]. This is written as $G_j$. The string G[j...l] is called prefix of $G_j$. The suffix array SA[1,...,n] is an array including an integer j corresponding to Gj. The prefixes are sorted in dictionary order (such as in alphabetical order in this example). When a length of the longest common prefix between the strings s and t is |lcp(s,t)|, a height array Hgt[1,...,N] is defined as:

$$Hgt[i]=|lcp(T_{SA[i]},T_{SA[i+1]}|$$

Here, Hgt[1]=0 is defined. By using this array, a length causing the incidence of the prefix of $T_{SA[i]}$ to be "1" for the first time in the string G can be obtained as:

$$maxHgt[i]=1+\max\{Hgt[i-1],Hgt[i]\}$$

where the length is maxHgt[i]. Here, maxHtg[1]=1+Hgt [1].

[Technique of Calculating Isolation Degree]

An isolation degree of the partial array E with respect to the genome array G can be obtained by scanning G only once. The calculation requires a period of time, $O(|G|(|E|\log|E|)^{1/2})$. When the maximum number k of mismatches is given, the calculation time is $O(|G|(k\log k)^{1/2})$. The inventors know that the second isolation degree isoln(E,G) can be calculated in a period of time, $O(|G|(|E|\log|E|)^{1/2})$. However, for a human genome, |G| has a size of about $3\times10^9$. Therefore, more reduction of the calculation time is required.

Therefore, the inventors invented to calculate the lower bound of the isolation degree of a given array E with respect to G by using a sub-table storing isolation degrees of short partial arrays as many as a memory could hold.

[Introduction of Divided String]

A division dec(E,L) of an array E is defined as a set of partial arrays resulting from the division of the array E into m such that the lengths of the partial arrays can be uniquely Li(where i=1, ..., m).

(1)

The $i^{th}$ partial array is defined as $dec_i(E,L)$.

The inventors found that, when an array E was given, the following equation held for a given division dec(E,L).

(2)

Furthermore, the system holds a table of isolation degrees relating to partial arrays having lengths p (such as 18 mer) and below. Here, the following equation holds from the equation above.

(3)

where the equal signs hold when p=|E|.

In order to calculate the left side of the inequality, the following technique can be adopted.

A function f(E) is defined as:

(4)

Based on this, the following linear recurrence equation can be obtained, and a lower bound f(E) of the isolation degree can be calculated for a period of time O(|E||p|).

$$f(E_{[1,i]})=isol_n(E_{[1,i]},G) \text{ (where i=1, ..., p)}$$

$$f(E_{[1,i]})=max\{f(E_{[1,i-j]})$$

$$+isol_n(E_{[i-j+1,i]},G)|j=1, ..., p\}$$

(a recursive step where i>p)

By solving the recurrence equation about an array E, the isolation degree $isol_n(E,G)$ can be obtained. Furthermore, when $isol_n(E_{[1,i]},G)$ (where i=1, ..., p) can be calculated for a constant time by using a sub-table, which will be described below, the recurrence equation above can be calculated for a period of time O(|E||P|).

[Sub-Table]

In order to calculate the lower bound of the isolation degree of the array E, all isolation degrees of partial arrays having a length |p| and below must be calculated. According to this embodiment, a suffix array and a height array are used. While the maximum height array maxHgt[i] has been described above, this can be regarded as a length by which the incidence of the $i^{th}$ prefix in the suffix array is one or below in the array G for the first time. Extending the definition of the maximum height array, the maximum height array is defined as "a length by which the incidence of the $i_{th}$ prefix of a given suffix array is k or below in the array G for the first time", $maxHgt_k[i]$. In order to calculate the maximum height array, the definition of a height array is extended as:

$$Hgt_k[i]=|lcp(T_{SA[i]},T_{SA[i+k]})|$$

By using the height array $hgt_k$, the maximum height array $maxHgt_k[i]$ can be obtained.

$$maxHgt_k[i]=1+max\{[Hgt_k[i-j]|j=0, ..., k\}$$

When a data structure is used in which the number of elements under each node of a suffix tree is written in the node, the maximum height array $maxHgt_k$ can be calculated for a period of time O(|G|) by making the round of a tree in a depth-oriented manner. However, since 16n bytes are required for storing a suffix tree, a memory capacity of 48G bytes is required for storing a suffix tree of a human genome array (3 G(giga)bytes). Since 6 bytes are required for storing each node in which the number of leaves under the node of the suffix tree is limited to $2^8$ or below, 54 Gbytes are required in total. On the other hand, 4n bytes are required for a suffix array. A human genome array (3 G(giga) bytes) can be stored in 12 Gbytes. Even when a height array is stored for a length equal to or lower than $2^8$, only 15 Gbytes are required in total. Therefore, a suffix array is desirably used in consideration of the memory capacity.

By using the maximum height array $maxHgt_k$, a partial array E with a length 1 starting from a position i on the genome array G and an isolation degree $isol_k$ (E,G) thereof can be categorized as:

$$isol_k(E,G) = 0 \ (1<maxHgt_k[SA[i]])$$

$$= 1 \ (1=maxHgt_k[SA[i]])$$

$$\geq 1 \ (1>maxHgt_k[SA[i]])$$

All of the isolation degrees $isol_k(E,G)$ of the partial array E having a length |p| and below starting from all positions in the genome array G must be calculated. However, for $maxHgt_n[SA[i]]\geq|p|$, the isolation degree is "0" or "1". Thus, a constant time can be obtained from the maximum height array $maxHgt_k$ and the suffix array. In order to calculate a maximum height accurately for all of the partial arrays E having a length |p| and below, a separate calculation must be performed separately for $maxHgt_k[SA[i])<|P|$. However, the step can be omitted in consideration of the calculation of a lower bound of the isolation degrees.

When the accurate calculation of the maximum height is not performed, a length |p| is desirably used by which the isolation degree $isol_k(E,G)$ of partial arrays E having the length |p| and below substantially agrees with the value resulting from the category above.

Fig. 8 is a graph showing a distribution of maximum height Hgt in a human genome array. In Fig. 8, the horizontal axis indicates the value of the maximum height array maxHgt while the vertical axis of the solid line graph indicates the number of a position in a human genome array where the maximum height is x. The vertical axis of the dotted like graph indicates a proportion of the number of positions in a human genome array with the maximum height equal to or lower than x in the entire arrays.

Referring to Fig. 8, when an array with a length of 16 (16 mer) is selected in a human genome array, the array may be unique (that is, the incidence is one) with the probability of 22%. On the other hand, an array with a length of 20 (20 mer) is unique with the probability of 74%. Apparently, using an incidence in an entire array as an indicator indicating uniqueness thereof is not appropriate for arrays with a length of 20 mer or higher.

Referring to Fig. 8, most arrays congregate at the maximum height of 17 mer. Here, when a length whereby the maximum height is "2" for the first time is calculated, the pattern peak may be estimated as being with a length of 18 mer or more. A part of those having the isolation degree at "0" or "1" can be accurately calculated by using $maxHgt_k$. Thus, a desired length |p| is achieved when the isolation degrees of most arrays having the length |p| or below are "0" or "1". Therefore, |p|=18 is the most suitable for a human genome array. In order to search |p|, a lower bound is calculated by moving p in a recurrence equation in the range of 1<|p|<|E|. However, the lower bound is calculated from $maxHgt_k$ without accurately calculating the isolation degrees with the length |p|. This technique is equivalent to solving the above-described recurrence equation by using a value obtained from $maxHgt_k$ with p=|E|. Though the amount of time calculation of the lower bound is O ($E^2$), the calculation speed is sufficiently high in consideration of the fact that |E| is about 60 with $G>10^9$.

[Processing Example]

Fig. 9 is a flowchart showing an overview of processing to be performed in the design support apparatus according to the second embodiment. As shown in Fig. 9, a maximum height arrays ($maxHgt_1(i)$, $maxHgt_2(i)$, ...) are first calculated in the design support apparatus (step 901). For example, $maxHgt_1(i)$ and $maxHgt_2(i)$ can be obtained from the following equation. As described above, $maxHgt_k(i)$ refers to a maximum length by which a string (partial array) starting from the position i occurs k times or below in the genome array G for the first time.

$$maxHgt_1(i)=$$

$$\max(|lcp(i,i+1)|, |lcp(i,i-1)|+1$$

$$maxHgt_2(i)=$$

$$\max(|lcp(i,i+2)|, |lcp(i-1,i+1)|, |lcp(i-2,i)|+1$$

Next, a table is prepared having a calculation result of the second isolation degree of a partial array having a length |p| (such as |p|=18) or below (step 902). Then, by using the above-described recurrence equation,

$$f(E_{[1,i]})=isol_n(E_{[1,i]},G) \text{ (where i=1, ..., p)}$$

$$f(E_{[1,i]})=\max\{f(E_{[1,i-j]})$$

$$+isol_n(E_{[i-j+1,i]},G)|j=1, ..., p\}$$

(a recursive step where i>p),

the lower bound f(E) is calculated (step 903).

Industrial Applicability

The invention is applicable to support to design oligonucleotide arrays using a unique array, for example. The unique array can be obtained from a large amount of array information (such as human genome arrays), which may be useful for PCR primer design, design of microarray oligonucleotide, array design for RNAi, array design for genetic screening and array design for genome typing.

**Claims**

1. A method for calculating an indicator indicating an incidence of an array in a genome array, the method **characterized by** comprising the steps of:

   calculating incidences of partial arrays with a predetermined length in the genome array; and storing the incidences relating to the partial arrays with the predetermined length in an incidence table.

2. A method according to Claim 1, **characterized by** that the step of storing in the incidence table has the steps of:

   omitting the storage into the incidence table for partial arrays with the incidence of zero (0); and using second partial arrays having a shorter second predetermined length than the predetermined length and storing in a second table a position in the incidence table of the partial arrays with the predetermined length including the second partial array from the beginning.

3. A method for calculating an indicator indicating an isolation degree of an array in a genome array, the method **characterized by** comprising the steps of:

calculating an isolation degree i by which j mutation(s) (j=1,2, ..., i-1) referring to the conversion of j alkali(s) of each of partial arrays with a predetermined length do/does not appear in the genome array but i mutation (s) referring to the conversion of i alkalis appear(s) in the genome array; and
storing in an isolation degree table the isolation degree with respect to the partial arrays with the predetermined length.

**4.** A method according to Claim 3, **characterized by** that the step for calculating the isolation degree has the steps of:

judging whether or not k mutation(s) referring to the conversion of k alkali(s) of the partial array with the predetermined length exist(s) in the partial array with the predetermined length with reference to an incidence table storing an incidence in a genome array with respect to each of the partial arrays with the predetermined length;
when the k mutation(s) exist(s), determining k as an isolation degree;
when the k mutation(s) does/do not exist, incrementing k and repeating the step of judging the presence of the k mutation(s).

**5.** A method according to Claim 3 or 4 **characterized by** that the step of calculating the isolation degree has the steps of:

judging, by using second partial arrays having a shorter second predetermined length than the predetermined length and with reference to a second table storing a position, in the incidence table, of the partial arrays with the predetermined length including the second partial array from the beginning, whether the k mutation(s) with the predetermined length exist(s) in which k alkali(s) at a position away from the beginning of the partial array with the predetermined length by a second predetermined length is/are converted;
when the k mutation(s) exist(s), finding a hamming distance between the k mutation(s) and the array with the predetermined length;
when the minimum value of the hamming distance is k, determining the k as an isolation degree thereof;
when the minimum value is larger than k, repeating the step of incrementing k and judging by using the presence of the k mutation(s) with the predetermined length and the minimum value of the hamming distance.

**6.** A method according to Claim 3 or 4, **characterized by** comprising the step of judging the appearance in the genome array based on whether the incidence in the genome array is equal to or lower than n.

**7.** A method for calculating an indicator indicating an isolation degree of a genome array, the method **characterized by** comprising the steps of:

calculating a shortest partial array by which a partial array starting from the $k^{th}$ letter of a partial array with a predetermined length no longer appears in a genome array; and
calculating the maximum number m of partial array uniquely included in the partial array and handling the m as an indicator indicating an isolation degree thereof by considering the m as the lower bounds of the isolation degree.

**8.** A method according to Claim 7, **characterized by** comprising the step of performing the step of judging whether the partial array appears or not based on whether the incidence in the genome array is equal to or lower than n.

**9.** A method for calculating a first indicator indicating an eligibility for a primer of an array including a given alkali with respect to alkalis in a genome array by using an incidence table created by using a method according to Claim 1 or 2, the method **characterized by** comprising the steps of:

identifying a same number of arrays including the alkali as a predetermined length with respect to each of alkalis included in a genome array;
identifying an incidence relating to each of the identified arrays with reference to the incidence table; and
calculating the first indicator based on a total sum of the identified incidences.

**10.** A method for calculating a second indicator indicating an eligibility for a primer of an array including a given alkali with respect to alkalis in a genome array by using an isolation degree table created by using a method according to any one of Claims 3 to 8, the method **characterized by** comprising the steps of:

identifying a same number of arrays including the alkali as a predetermined length with respect to each of alkalis included in a genome array;
identifying an isolation degree relating to each of the identified arrays with reference to the isolation degree table; and
calculating the second indicator based on a total sum of the identified isolation degrees.

**11.** A method for calculating a third indicator indicating an eligibility, for a primer, of an array including a given alkali with respect to alkalis in a genome array by using an incidence table created by using a method according to Claim 1 or 2 and an isolation degree table created by using a method according to any one of Claims 3 to 8, the method **characterized by** comprising the steps of:

identifying a same number of arrays including the alkali as a predetermined length with respect to each of alkalis included in a genome array;
identifying an incidence relating to each of the identified arrays with reference to the incidence table;
calculating a first indicator based on a total sum of the identified incidences;
identifying an isolation degree relating to each of the identified arrays with reference to the isolation degree table; and
calculating a second indicator based on a total sum of the identified isolation degrees.

**12.** A method **characterized by** comprising the steps of:

assigning, based on an indicator obtained by using a method according to any one of Claims 9 to 11, a different display form in accordance with a value or range of the indicator; and
creating an image representing each alkali in a genome array in accordance the assigned display form.

**13.** A method according to Claim 12, **characterized by** that the display form is a color.

**14.** A program for operating a computer for calculating an indicator indicating an incidence of an array in a genome array and being readable by the computer, the program causing the computer to perform the steps of:

calculating incidences of partial arrays with a predetermined length in the genome array; and
storing the incidences relating to the partial arrays with the predetermined length in an incidence table.

**15.** A program according to Claim 14, **characterized by** causing the computer to perform the step for storing in the incidence table having the steps of:

omitting the storage into the incidence table for partial arrays with the incidence of zero (0); and
using second partial arrays having a shorter second predetermined length than the predetermined length and storing in a second table a position, in the incidence table, of the partial arrays with the predetermined length including the second partial array from the beginning.

**16.** A program for operating a computer for calculating an indicator indicating an isolation degree of an array in a genome array and being readable by the computer, the program causing the computer to perform the steps of:

calculating an isolation degree i by which j mutation(s) (j=1,2, ..., i-1) referring to the conversion of j alkali(s) of each of partial arrays with a predetermined length do/does not appear in the genome array but i mutation(s) referring to the conversion of i alkalis appear(s) in the genome array; and
storing in an isolation degree table the isolation degree with respect to the partial arrays with the predetermined length.

**17.** A program according to Claim 16, **characterized by** causing the computer to perform the step for calculating the isolation degree having the steps of:

judging whether or not k mutation(s) referring to the conversion of k alkali(s) of the partial array with the predetermined length exist(s) in the partial array with the predetermined length with reference to an incidence table storing an incidence in a genome array with respect to each of the partial arrays with the predetermined length;
when the k mutation(s) exist(s), determining the k as an isolation degree;
when the k mutation(s) does/do not exist, incrementing k and repeating the step of judging the presence of the k mutation(s).

**18.** A program according to Claim 16 or 17 **characterized by** the program causing the computer to perform the step of calculating the isolation degree having the steps of:

judging, by using second partial arrays having a shorter second predetermined length than the predetermined length and with reference to a second table storing a position in the incidence table of the partial arrays with the predetermined length including the second partial array from the beginning, whether or not the k mutation(s) with the predetermined length exist(s) in which k alkali(s) at a position away from the beginning of the partial array with the predetermined length by a second predetermined length is/are converted;
when the k mutation(s) exist(s), finding a hamming distance between the k mutation(s) and the array with the predetermined length;
when the minimum value of the hamming distance is k, determining the k as an isolation degree thereof;
when the minimum value is larger than k, repeating the step of incrementing k and judging by using the presence of the k mutation(s) with the predetermined length and the minimum value of the hamming distance.

**19.** A program according to Claim 16 or 17, **characterized by** causing the computer to perform the step of judging the appearance in the genome array based on whether the incidence in the genome array is equal to or lower than n.

**20.** A program for operating a computer for calculating an indicator indicating an isolation degree of a genome array and being readable by the computer, the program causing the computer to perform the steps of:

calculating a shortest partial array by which a partial array starting from the $k^{th}$ letter of a partial array with a predetermined length no longer appears in a genome array; and
calculating the maximum number m of partial array uniquely included in the partial array and handling the m as an indicator indicating an isolation degree thereof by considering the m as the lower bounds of the isolation degree.

**21.** A program according to Claim 20, **characterized by** causing the computer to perform the step of judging whether the partial array appears or not based on whether the incidence in the genome array is equal to or lower than n.

**22.** A computer-readable program for operating a computer for calculating a first indicator indicating an eligibility for a primer of an array including a given alkali with respect to alkalis in a genome array by using an incidence table created by causing the computer to perform a program according to Claim 14 or 15, the method **characterized by** causing the computer to perform the steps of:

identifying a same number of arrays including the alkali as a predetermined length with respect to each of alkalis included in a genome array;
identifying an incidence relating to each of the identified arrays with reference to the incidence table; and
calculating a first indicator based on a total sum of the identified incidences.

**23.** A computer-readable program for operating a computer for calculating a second indicator indicating an eligibility, for a primer, of an array including a given alkali with respect to alkalis in a genome array by using an isolation degree table created by causing the computer to perform a program according to any one of Claims 16 to 21, **characterized by** causing the computer to perform the steps of:

identifying a same number of arrays including the alkali as a predetermined length with respect to each of alkalis included in a genome array;
identifying an isolation degree relating to each of the identified arrays with reference to the isolation degree table; and
calculating the second indicator based on a total sum of the identified isolation degrees.

**24.** A computer-readable program for operating a computer for calculating a third indicator indicating an eligibility for a primer of an array including a given alkali with respect to alkalis in a genome array by using an incidence table created by causing the computer to perform a program according to Claim 14 or 15 and an isolation degree table created by causing the computer to perform a program according to any one of Claims 16 to 21, **characterized by** causing the computer to perform the steps of:

identifying a same number of arrays including the alkali as a predetermined length with respect to each of alkalis included in a genome array;
identifying an incidence relating to each of the identified arrays with reference to the incidence table;
calculating a first indicator based on a total sum of the identified incidences;
identifying an isolation degree relating to each of the identified arrays with reference to the isolation degree table; and
calculating a second indicator based on a total sum of the identified isolation degrees.

**25.** A program **characterized by** causing the computer to perform the steps of:

assigning, based on an indicator obtained by causing the computer to perform a program according to any one of Claims 22 to 24, a different display form in accordance with a value or range of the indicator; and
creating an image representing each alkali in a genome array in accordance the assigned display form.

**Amended claims under Art. 19.1 PCT**

**1.** (Amended) A method for judging an eligibility, for array design, of an array including an alkali in a genome array, the method **characterized by** comprising the steps of:

calculating incidences of partial arrays with a predetermined length in the genome array; and
storing the incidences relating to the partial arrays with the predetermined length in an incidence table.

**2.** A method according to Claim 1, **characterized by** that the step of storing in the incidence table has the steps of:

omitting the storage into the incidence table for partial arrays with the incidence of zero (0); and
using second partial arrays having a shorter second predetermined length than the predetermined length and storing in a second table a position in the incidence table of the partial arrays with the predetermined length including the second partial array from the beginning.

**3.** (Amended) A method for judging an eligibility, for array design, of an array including an alkali in a genome array, the method **characterized by** comprising the steps of:

calculating an isolation degree i by which j mutation(s) (j=1,2, ,,,, i-1) referring to the conversion of j alkali(s) of each of partial arrays with a predetermined length do/does not appear in the genome array but i mutation(s) referring to the conversion of i alkalis appear(s) in the genome array; and
storing in an isolation degree table the isolation degree with respect to the partial arrays with the predetermined length.

**4.** A method according to Claim 3, **characterized by** that the step for calculating the isolation degree has the steps of:

judging whether or not k mutation(s) referring to the conversion of k alkali(s) of the partial array with the predetermined length exist(s) in the partial array with the predetermined length with reference to an incidence table storing an incidence in a genome array with respect to each of the partial arrays with the predetermined length;
when the k mutation(s) exist(s), determining k as an isolation degree;
when the k mutation(s) does/do not exist, incrementing k and repeating the step of judging the presence of the k mutation(s).

**5.** A method according to Claim 3 or 4 **characterized by** that the step of calculating the isolation degree has the steps of:

judging, by using second partial arrays having a shorter second predetermined length than the predetermined length and with reference to a second table storing a position, in the incidence table, of the partial arrays with the predetermined length including the second partial array from the beginning, whether the k mutation(s) with the predetermined length exist(s) in which k alkali(s) at a position away from the beginning of the partial array with the predetermined length by a second predetermined length is/are converted;
when the k mutation(s) exist(s), finding a hamming distance between the k mutation(s) and the array with the predetermined length;
when the minimum value of the hamming distance is k, determining the k as an isolation degree thereof;
when the minimum value is larger than k, repeating the step of incrementing k and judging by using the presence of the k mutation(s) with the predetermined length and the minimum value of the hamming distance.

**6.** A method according to Claim 3 or 4, **characterized by** comprising the step of judging the appearance in the genome array based on whether the incidence in the genome array is equal to or lower than n.

**7.** (Amended) A method for judging an eligibility for array design of an array including an alkali in a genome array, the method **characterized by** comprising the steps of:

calculating a shortest partial array by which a partial array starting from the $k^{th}$ letter of a partial array with a predetermined length no longer appears in a genome array; and
calculating the maximum number m of partial array uniquely included in the partial array and handling the m as an indicator indicating an isolation degree thereof by considering the m as the lower bounds of the isolation degree.

**8.** A method according to Claim 7, **characterized by** comprising the step of performing the step of judging whether the partial array appears or not based on whether the incidence in the genome array is equal to or lower than n.

**9.** (Amended) A method for judging an eligibility, for array design, of an array including an alkali in a genome array, the method **characterized by** comprising the steps of:

creating an incidence table by using a method according to Claim 1 or 2;
identifying a same number of arrays including the alkali as a predetermined length with respect to each of alkalis included in a genome array;
identifying an incidence relating to each of the identified arrays with reference to the incidence table; and
calculating the first indicator based on a total sum of the identified incidences.

**10.** (Amended) A method for judging an eligibility, for array design, of an array including an alkali in a genome array, the method **characterized by** comprising the steps of:

using an isolation degree table created by using a method according to any one of Claims 3 to 8;
identifying a same number of arrays including the alkali as a predetermined length with respect to each of alkalis included in a genome array;
identifying an isolation degree relating to each of the identified arrays with reference to the isolation degree table; and
calculating the second indicator based on a total sum of the identified isolation degrees.

**11.** (Amended) A method for judging an eligibility, for array design, of an array including an alkali in a genome array, **characterized by** comprising the steps of:

using an incidence table created by using a method according to Claim 1 or 2;
using an isolation degree table created by using a method according to any one of Claims 3 to 8;
identifying a same number of arrays including the alkali as a predetermined length with respect to each of alkalis included in a genome array;
identifying an incidence relating to each of the identified arrays with reference to the incidence table;
calculating a first indicator based on a total sum of the identified incidences;
identifying an isolation degree relating to each of the identified arrays with reference to the isolation degree table; and
calculating a second indicator based on a total sum of the identified isolation degrees.

**12.** (Amended) A method according to any one of Claims 9 to 11 **characterized by** further comprising the steps of:

assigning, based on an calculated indicator, a different display form in accordance with a value or range of the indicator; and
creating an image representing each alkali in a genome array in accordance the assigned display form.

**13.** A method according to Claim 12, **characterized by** that the display form is a color.

**14.** (Amended) A program for operating a computer for judging an eligibility for array design of an array including an alkali in a genome array and being readable by the computer, the program **characterized by** causing the computer to perform the steps of:

calculating incidences of partial arrays with a predetermined length in the genome array; and
storing the incidences relating to the partial arrays with the predetermined length in an incidence table.

**15.** A program according to Claim 14, **characterized by** causing the computer to perform the step for storing in the incidence table having the steps of:

omitting the storage into the incidence table for partial arrays with the incidence of zero (0); and
using second partial arrays having a shorter second predetermined length than the predetermined length and storing in a second table a position, in the incidence table, of the partial arrays with the predetermined length including the second partial array from the beginning.

**16.** (Amended) A program for operating a computer for judging an eligibility, for array design, of an array including an alkali in a genome array and being readable by the computer, the program **characterized by** causing the computer to perform the steps of:

calculating an isolation degree i by which j mutation (s) (j=1,2, ..., i-1) referring to the conversion of j alkali(s) of each of partial arrays with a predetermined length do/does not appear in the genome array but i mutation(s) referring to the conversion of i alkalis appear(s) in the genome array; and
storing in an isolation degree table the isolation degree with respect to the partial arrays with the predetermined length.

**17.** A program according to Claim 16, **character-**

**ized by** causing the computer to perform the step for calculating the isolation degree having the steps of:

judging whether or not k mutation(s) referring to the conversion of k alkali(s) of the partial array with the predetermined length exist(s) in the partial array with the predetermined length with reference to an incidence table storing an incidence in a genome array with respect to each of the partial arrays with the predetermined length;
when the k mutation(s) exist(s), determining the k as an isolation degree;
when the k mutation(s) does/do not exist, incrementing k and repeating the step of judging the presence of the k mutation(s).

**18.** A program according to Claim 16 or 17 **characterized by** the program causing the computer to perform the step of calculating the isolation degree having the steps of:

judging, by using second partial arrays having a shorter second predetermined length than the predetermined length and with reference to a second table storing a position, in the incidence table, of the partial arrays with the predetermined length including the second partial array from the beginning, whether or not the k mutation(s) with the predetermined length exist(s) in which k alkali(s) at a position away from the beginning of the partial array with the predetermined length by a second predetermined length is/are converted;
when the k mutation(s) exist(s), finding a hamming distance between the k mutation(s) and the array with the predetermined length;
when the minimum value of the hamming distance is k, determining the k as an isolation degree thereof;
when the minimum value is larger than k, repeating the step of incrementing k and judging by using the presence of the k mutation(s) with the predetermined length and the minimum value of the hamming distance.

**19.** A program according to Claim 16 or 17, **characterized by** causing the computer to perform the step of judging the appearance in the genome array based on whether the incidence in the genome array is equal to or lower than n.

**20.** (Amended) A program for operating a computer for judging an eligibility for array design of an array including an alkali in a genome array and being readable by the computer, the program **characterized by** causing the computer to perform the steps of:

calculating a shortest partial array by which a partial array starting from the kth letter of a partial array with a predetermined length no longer appears in a genome array; and
calculating the maximum number m of partial array uniquely included in the partial array and handling the m as an indicator indicating an isolation degree thereof by considering the m as the lower bounds of the isolation degree.

**21.** A program according to Claim 20, **characterized by** causing the computer to perform the step of judging whether the partial array appears or not based on whether the incidence in the genome array is equal to or lower than n.

**22.** (Amended) A computer-readable program for operating a computer for judging an eligibility for array design of an array including an alkali in a genome array and being readable by the computer, the program **characterized by** causing the computer to perform the steps of:

using an incidence table created by causing the computer to perform a program according to Claim 14 or 15,
identifying a same number of arrays including the alkali as a predetermined length with respect to each of alkalis included in a genome array;
identifying an incidence relating to each of the identified arrays with reference to the incidence table; and
calculating a first indicator based on a total sum of the identified incidences.

**23.** (Amended) A computer-readable program for operating a computer for judging an eligibility, for array design, of an array including an alkali in a genome array and being readable by the computer, the program **characterized by** causing the computer to perform the steps of:

using an isolation degree table created by causing the computer to perform a program according to any one of Claims 16 to 21;
identifying a same number of arrays including the alkali as a predetermined length with respect to each of alkalis included in a genome array;
identifying an isolation degree relating to each of the identified arrays with reference to the isolation degree table; and
calculating the second indicator based on a total sum of the identified isolation degrees.

**24.** (Amended) A computer-readable program for operating a computer for judging an eligibility, for array design, of an array including an alkali in a genome array and being readable by the computer, the program **characterized by** causing the computer to perform the steps of:

> using an incidence table created by causing the computer to perform a program according to Claim 14 or 15; using an isolation degree table created by causing the computer to perform a program according to any one of Claims 16 to 21;
> identifying a same number of arrays including the alkali as a predetermined length with respect to each of alkalis included in a genome array;
> identifying an incidence relating to each of the identified arrays with reference to the incidence table;
> calculating a first indicator based on a total sum of the identified incidences;
> identifying an isolation degree relating to each of the identified arrays with reference to the isolation degree table;
> calculating a second indicator based on a total sum of the identified isolation degrees.

**25.** (Amended) A program according to any one of Claims 22 to 24 **characterized by** further causing the computer to perform the steps of:

> assigning, based on an calculated indicator, a different display form in accordance with a value or range of the indicator; and
> creating an image representing each alkali in a genome array in accordance the assigned display form.

FIG. 1

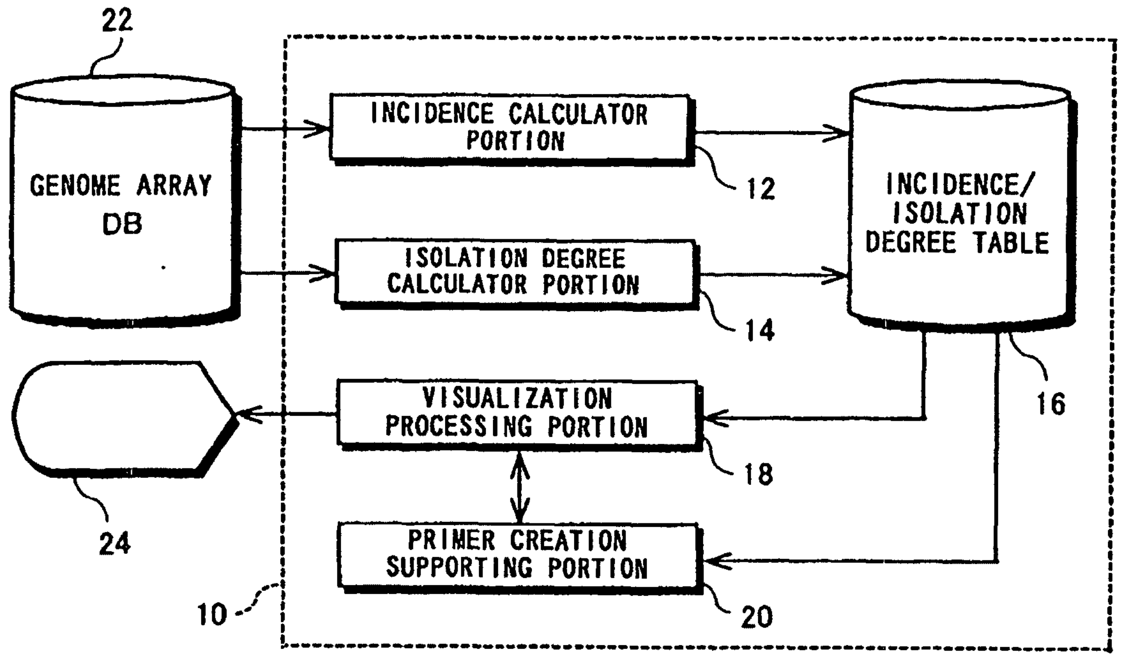


FIG. 7

| FIRST INDICATOR (INCIDENCES) | SECOND INDICATOR (ISOLATION DEGREE) | THIRD INDICATOR (SECOND INDICATOR / FIRST INDICATOR) | COLOR |
|---|---|---|---|
| LARGE<br>↕<br>SMALL | SMALL<br>↕<br>LARGE | SMALL<br>↕<br>LARGE | WARM COLOR<br>↕<br>COLD COLOR |

# FIG. 2A

200

| GENOME ARRAYS: | ATATGGGATC | INCIDENCES |
|---|---|---|
| N-mer ARRAY: | AT | AT:3 |
| | TA | TA:1 |
| | AT | AT:3 |
| | TG | TG:1 |
| | GG | GG:2 |
| | GG | GG:2 |
| | GA | GA:1 |
| | AT | AT:3 |
| | TC | TC:1 |

# FIG. 2B

211

210

| ···ATGCCAGTCAG··· | INCIDENCES |
|---|---|
| ATGCCA | 8 |
| TGCCAG | 2 |
| GCCAGT | 3 |
| CCAGTC | 1 |
| CAGTCA | 3 |
| AGTCAG | 4 |

212

APPEARS (8+2+3+1+3+4)/6 TIMES

# FIG. 3

300 301 302 303 304

| ATATGGGATC | INCIDENCES | 1 MUTATION | 2 MUTATIONS | ISOLATION DEGREE |
|---|---|---|---|---|
| AT | AT:3 | AA,AG,AC,TT,GT,CT | TA,TG,TC,GA,GG,GC,CA,CG,CC | 2 |
| TA | TA:1 | AA,GA,CA,TT,TG,TC | | 1 |
| AT | AT:3 | AA,AG,AC,TT,GT,CT | TA,TG,TC,GA,GG,GC,CA,CG,CC | 2 |
| TG | TG:1 | TA,TC,TT,AG,CG,GG | | 1 |
| GG | GG:2 | GA,GC,GT,AG,CG,TG | | 1 |
| GG | GG:2 | GA,GC,GT,AG,CG,TG | | 1 |
| GA | GA:1 | GG,GC,GT,AA,CA,TA | | 1 |
| AT | AT:3 | AA,AG,AC,TT,GT,CT | TA,TG,TC,GA,GG,GC,CA,CG,CC | 2 |
| TC | TC:1 | TA,TG,TT,AC,GC,CC | | 1 |

FIG. 4

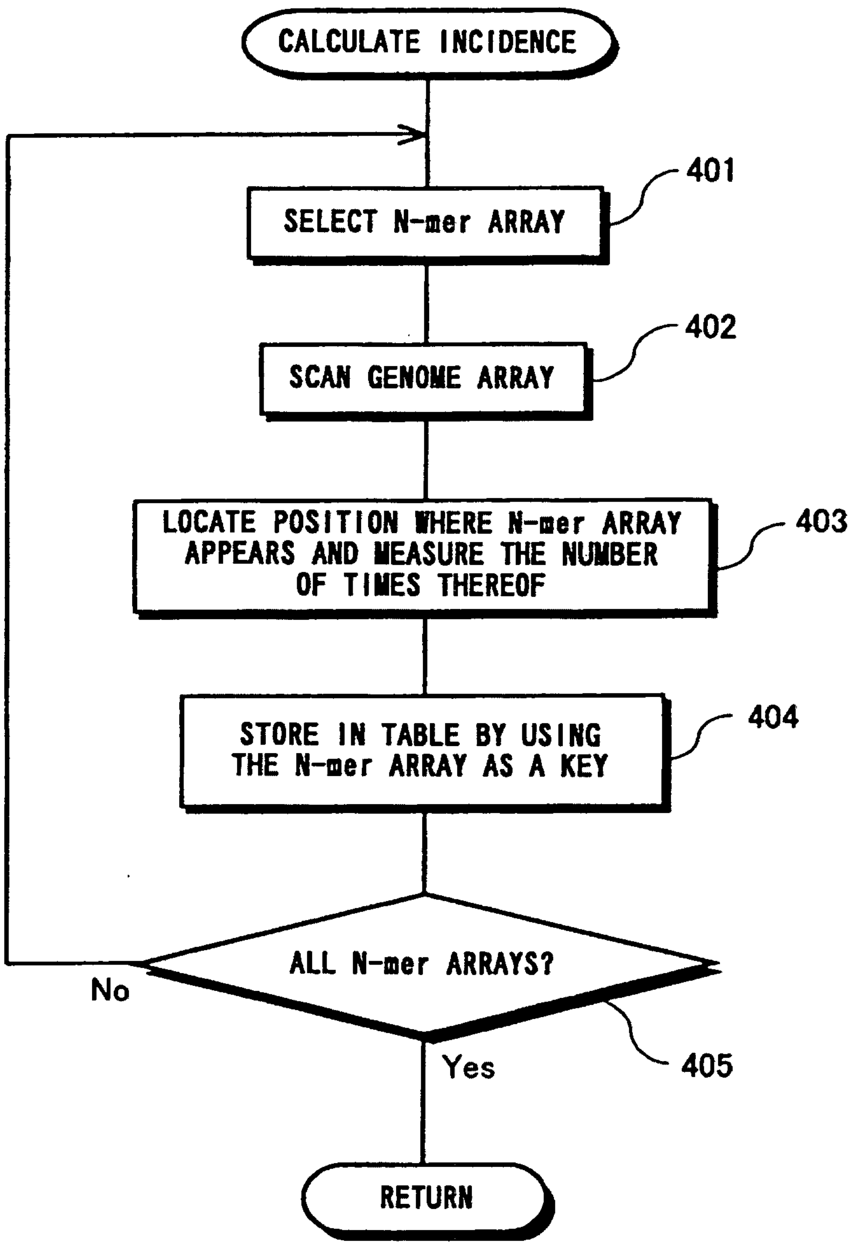
CALCULATE INCIDENCE
SELECT N-mer ARRAY
401
SCAN GENOME ARRAY
402
LOCATE POSITION WHERE N-mer ARRAY APPEARS AND MEASURE THE NUMBER OF TIMES THEREOF
403
STORE IN TABLE BY USING THE N-mer ARRAY AS A KEY
404
ALL N-mer ARRAYS?
No
Yes
405
RETURN

FIG. 5A

| N-mer ARRAYS | TT | TC | TG | TA | CT | CC | CG | CA | GT | GC | GG | GA | AT | AC | AG | AA |
|---|---|---|---|---|---|---|---|---|---|---|---|---|---|---|---|---|
| INCIDENCES | 0 | 1 | 1 | 1 | 0 | 0 | 0 | 0 | 0 | 0 | 2 | 1 | 3 | 0 | 0 | 0 |

FIG. 5B

501

| N-mer ARRAYS | TC | TG | TA | GG | GA | AT |
|---|---|---|---|---|---|---|
| INCIDENCES | 1 | 1 | 1 | 2 | 1 | 3 |

FIG. 5C

502

| HASH ARRAYS | T | C | G | A |
|---|---|---|---|---|
| POSITION OF APPEARANCE | 1 | 0 | 4 | 6 |

FIG. 6

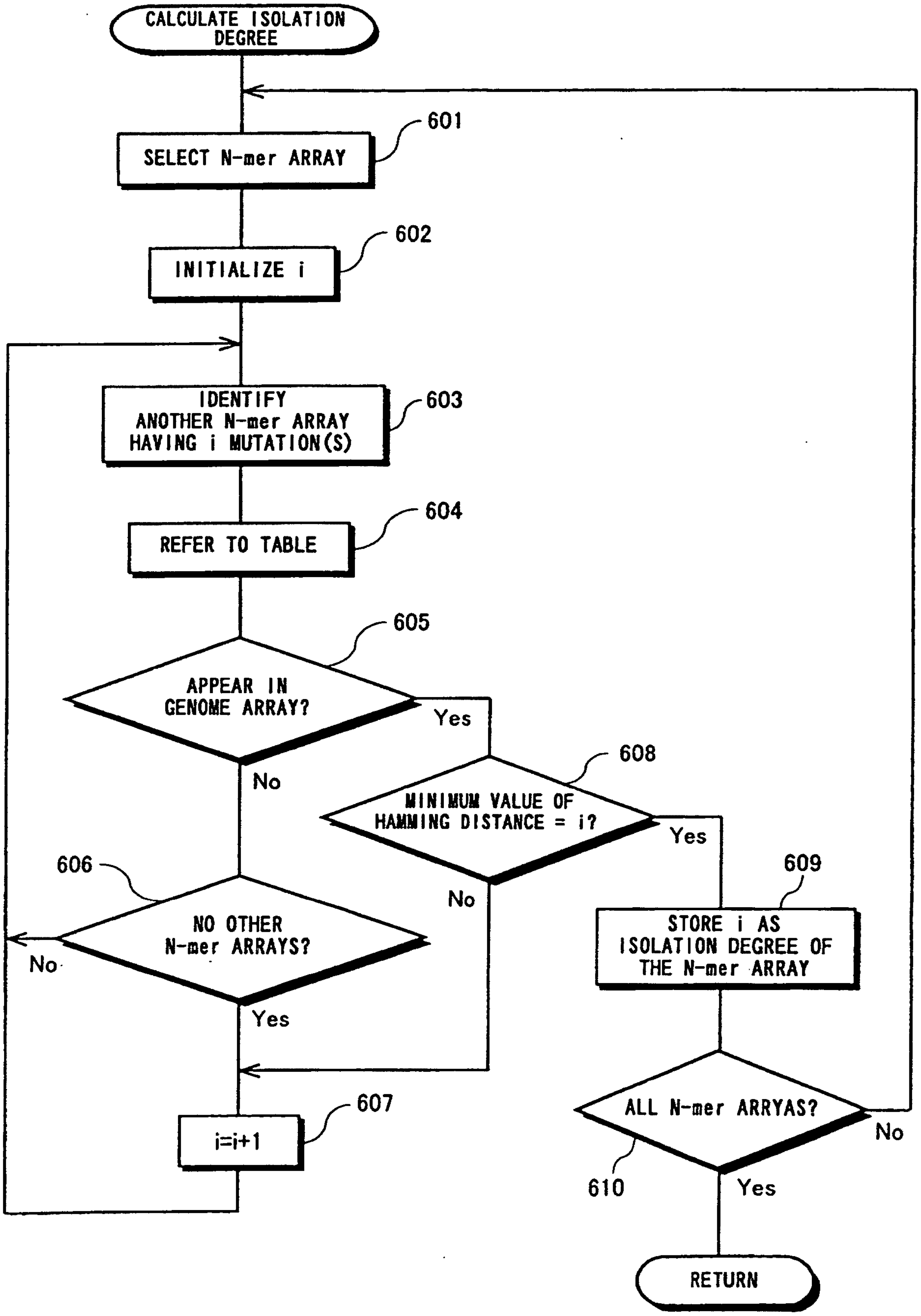
CALCULATE ISOLATION DEGREE
601
SELECT N-mer ARRAY
602
INITIALIZE i
603
IDENTIFY ANOTHER N-mer ARRAY HAVING i MUTATION(S)
604
REFER TO TABLE
605
APPEAR IN GENOME ARRAY?
Yes
No
608
MINIMUM VALUE OF HAMMING DISTANCE = i?
Yes
No
606
NO OTHER N-mer ARRAYS?
No
Yes
609
STORE i AS ISOLATION DEGREE OF THE N-mer ARRAY
607
i=i+1
ALL N-mer ARRYAS?
No
610
Yes
RETURN

FIG. 8

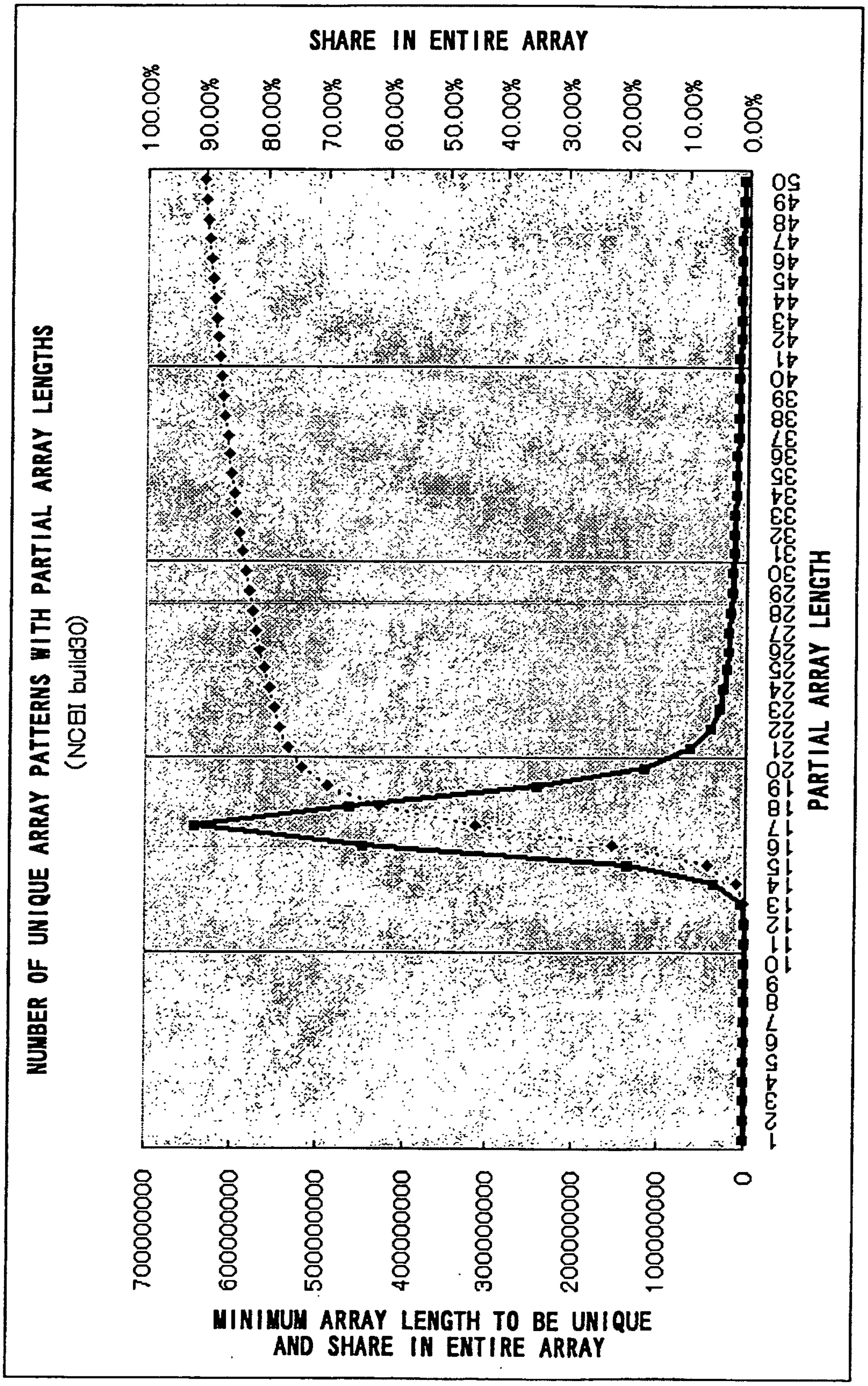
NUMBER OF UNIQUE ARRAY PATTERNS WITH PARTIAL ARRAY LENGTHS
(NCBI build30)
SHARE IN ENTIRE ARRAY
100.00%
90.00%
80.00%
70.00%
60.00%
50.00%
40.00%
30.00%
20.00%
10.00%
0.00%
700000000
600000000
500000000
400000000
300000000
200000000
100000000
0
PARTIAL ARRAY LENGTH
MINIMUM ARRAY LENGTH TO BE UNIQUE
AND SHARE IN ENTIRE ARRAY

FIG. 9

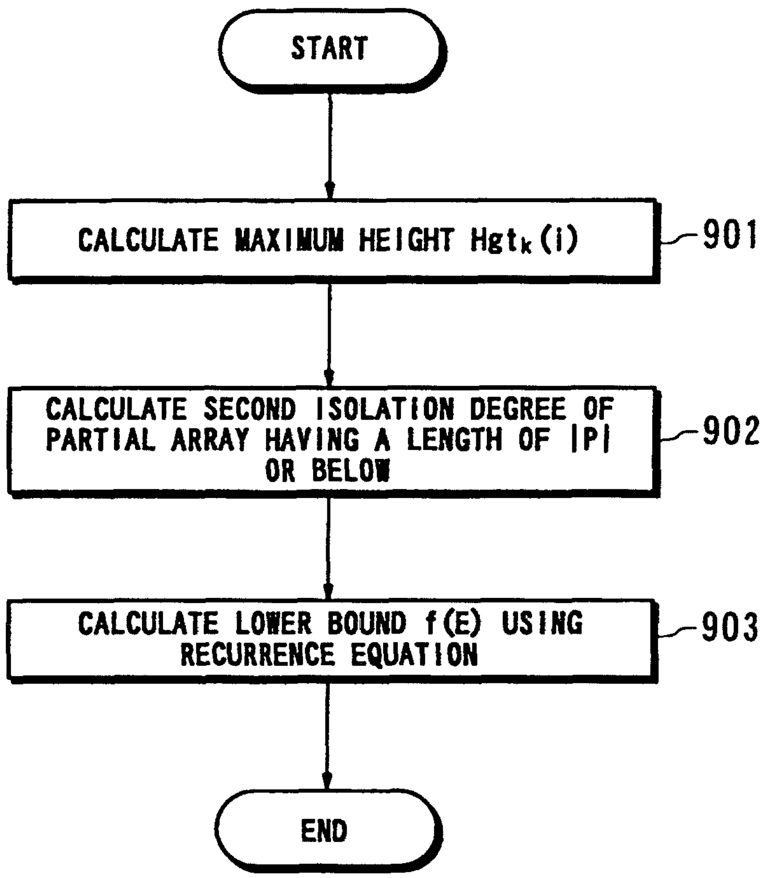
START
CALCULATE MAXIMUM HEIGHT $Hgt_k(i)$
901
CALCULATE SECOND ISOLATION DEGREE OF PARTIAL ARRAY HAVING A LENGTH OF |P| OR BELOW
902
CALCULATE LOWER BOUND f(E) USING RECURRENCE EQUATION
903
END

## INTERNATIONAL SEARCH REPORT

International application No.
PCT/JP02/13815

A. CLASSIFICATION OF SUBJECT MATTER
Int.Cl[7] G06F17/30, C12N15/00

According to International Patent Classification (IPC) or to both national classification and IPC

B. FIELDS SEARCHED

Minimum documentation searched (classification system followed by classification symbols)
Int.Cl[7] G06F17/30, C12N15/00

Documentation searched other than minimum documentation to the extent that such documents are included in the fields searched

| | | | |
|---|---|---|---|
| Jitsuyo Shinan Koho | 1922-1996 | Toroku Jitsuyo Shinan Koho | 1994-2003 |
| Kokai Jitsuyo Shinan Koho | 1971-2003 | Jitsuyo Shinan Toroku Koho | 1996-2003 |

Electronic data base consulted during the international search (name of data base and, where practicable, search terms used)
JICST FILE(JOIS), WPI, INSPEC(DIALOG)

C. DOCUMENTS CONSIDERED TO BE RELEVANT

| Category* | Citation of document, with indication, where appropriate, of the relevant passages | Relevant to claim No. |
|---|---|---|
| X<br>Y | JP 7-105224 A (Hitachi, Ltd.),<br>21 April, 1995 (21.04.95),<br>Claim 1<br>(Family: none) | 1,14<br>2,15 |
| Y | JP 4-274557 A (Hitachi, Ltd.),<br>30 September, 1992 (30.09.92),<br>Par. Nos. [0023], [0024]<br>& WO 90/16036 A & EP 437615 A<br>& EP 468402 A & EP 501416 A<br>& ES 523700 A & EP 561364 A<br>& US 5138669 A & US 5140644 A<br>& US 5168533 A & US 5220625 A<br>& US 5454105 A & US 5469354 A<br>& US 5471610 A & US 5519857 A<br>& US 5748953 A & US 5757983 A<br>& US 6094647 A | 2,15 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 April, 2003 (14.04.03) | 30 April, 2003 (30.04.03) |
| Name and mailing address of the ISA/<br>Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP02/13815

C (Continuation). DOCUMENTS CONSIDERED TO BE RELEVANT

| Category* | Citation of document, with indication, where appropriate, of the relevant passages | Relevant to claim No. |
|---|---|---|
| Y | FUKUSHIMA et al., "Zenbun Kensakuyo Moji Seibunhyo no Ichi Asshuku Hoshiki", Information Processing Society of Japan Dai 47 Kai Zenkoku Taikai Koen Ronbunshu (4), 06 October, 1993 (06.10.93), pages 83 to 84, particularly, Fig. 1 | 2,15 |
| Y | FUKUSHIMA et al., "Kosoku Zenbun Kensaku no tame no Flexible Mojiretsu Inversion-ho (1) Hoshiki Gaiyo", Joho Shori, 15 April, 1997 (15.04.97), Vol.38, No.4, pages 334 to 335, particularly, Fig. 1 | 2,15 |
| A | MIYAKE et al., "Kakusan Hairetsu Bunseki eno Fuzzy Matching Kansu no Oyo", Dai 6 Kai Fuzzy System Symposium Koen Ronbunshu, 06 September, 1990 (06.09.90), pages 307 to 310, particularly, Fig. 3 | 1,2,14,15 |

Form PCT/ISA/210 (continuation of second sheet) (July 1998)

**INTERNATIONAL SEARCH REPORT**

International application No.
PCT/JP02/13815

**Box I Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

Claims 1, 2, 14 and 15 relate to the calculation of an occurrence frequency.
Claims 3 to 6 and 16 to 19 relate to the calculation of a degree of isolation considering mutation.
Claims 7, 8, 20 and 21 relate to the calculation of a degree of isolation considering the length of a partial sequence.
Claims 9 and 22 relate to the calculation of a degree of adequacy for a primer referring to the frequency table.
Claims 10 and 23 relate to the calculation of a degree of adequacy for a primer referring to the degree of isolation table.
Claims 11 and 24 relate to the calculation (continued to extra sheet)

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.: 1, 2, 14 and 15

**Remark on Protest** ☐ The additional search fees were accompanied by the applicant's protest.
☐ No protest accompanied the payment of additional search fees.

Form PCT/ISA/210 (continuation of first sheet (1)) (July 1998)

**INTERNATIONAL SEARCH REPORT**

International application No.
PCT/JP02/13815

Continuation of Box No.II of continuation of first sheet(1)

of a degree of adequacy for a primer referring to the frequency table and the degree of isolation table.

Claims 12, 13 and 25 relate to the indication of a degree of adequacy for a primer.

These groups of inventions can be recognized neither as a single invention nor as relating to a group of inventions so linked as to form a single general inventive concept. Such being the case, claims 1 to 25 have seven groups of inventions.